# EUROPEAN PATENT APPLICATION

(11) **EP 1 872 774 A1**
(43) Date of publication of application: **02.01.2008**
(21) Application number: 06115066.0
(22) Date of filing: 07.06.2006
(51) Int. Cl.: A61K 9/00, A61K 47/48, A61K 31/568, A61K 31/565

(54) **A pharmaceutical composition adapted for oral transmucosal administration comprising sex hormone and its use**

(71) Applicant: Familplan Consulting Ltd Oy, 01531 Vantaa (FI)
(72) Inventor: Luukkainen, Tapani, FI-02160 Espoo (FI)
(74) Representative: Maskula, Silla Marjatta

(57) **Abstract**

The invention relates to a pharmaceutical composition adapted for oral transmucosal administration and its uses. The pharmaceutical composition comprises a water-soluble complex of a sex hormone and a cyclodextrin derivative, the amount of sex hormone in the composition being 0.01 - 7 mg.

## Description

The invention relates to a pharmaceutical composition adapted for oral transmucosal administration comprising a water-soluble complex of a sex hormone and a cyclodextrin derivative and its uses.

### BACKGROUND OF THE INVENTION

Human sex hormones have been identified and their role in the body functions has been studied extensively. It is known that supplementation with sex hormones, such as androgens and estrogens, has a potential to ameliorate some problems stemming from body wasting diseases and from aging.

However, numerous negative side effects prevent a wide use of such supplementations. When supplemented through gastrointestinal tract the hormones are rapidly metabolized by the liver, making a standard oral pill administration inefficient. This problem has been solved by the use of metabolism resisting unnatural analogs or parenteral depo formulations. However, some side effects still persist.

The level of sex hormones varies in healthy subjects. For example, in male mammals, testosterone is present in circulation at relatively low level at all times and small variations from the basal level occur in term of minutes. Additionally, large increases, called pulses, occur multiple times per hour or per day. The serum levels of sex hormones are regulated in a complex manner. Diseases, postnatal changes or aging may affect overall hormone levels.

The general belief is that for therapeutic purposes the level of the sex hormone in the body ought to be augmented to significantly high level. For example, widely used supplementation of testosterone is based on intramuscular injections of oily solution of testosterone esters, e.g. enanthate, from which testosterone is slowly released. This treatment strongly increases level of hormone for approximately a week, whereby it gradually decreases until the next injection is administered. Supplementations of steroid hormone by transdermal routes are administered in shorter intervals, but also these pharmaceutical forms supplies the hormone into circulation in considerable amounts raising the hormone level clearly above the natural basal level.

Sex hormones form water-soluble complexes with cyclodextrin derivatives. Cyclodextrins are oligosaccharides in which six, seven or eight glucose residues are joined into a cycle by glycosidic bonds. Beta-cyclodextrin has seven glucose residues. Derivatization of some of the hydroxyls of a cyclodextrin, for example, by methyl or hydroxypropyl groups, leads to mixtures, called cyclodextrin derivatives, which have, compared to the starting cyclodextrin, higher water solubility.

US 4,596,795 discloses administration of sex hormones in the form of hydrophilic cyclodextrin derivatives. It is stated that a daily dosage of 0.1 - 25 mg sex hormone can be administered by injecting by the buccal route, and sublingual administration can comprise 10 and 14 mg of testosterone cyclodextrin derivative complex. These high dosages elevate the hormone levels in serum for several hours over the basal level.

US 6,884,790 discloses a solid dosage form that does not disintegrate in water and contains a drug in form of inclusion complex with a cyclodextrin. It is stated that steroid hormones are typically administered in up to 10 mg doses.

### OBJECT OF THE INVENTION

The object of the invention is to minimise or even eliminate the problems existing in the prior art.

An object of the present invention is to provide a safe pharmaceutical composition for administering human sex hormones.

Another object of the invention is to minimise the adverse side effect associated with conventional sex hormone administration.

### SUMMARY OF THE INVENTION

In order to achieve the above-mentioned objects the present invention is characterised in what is defined in the characterising parts of the independent claims presented hereafter.

Typical pharmaceutical composition adapted for oral transmucosal administration according to the present invention comprises a water-soluble complex of a sex hormone and a cyclodextrin derivative, and the amount of sex hormone in the composition is 0.01 - 7 mg.

Typically, according to the present invention a complex of a sex hormone and a cyclodextrin derivative is used for manufacture of a oral transmucosal supplement for treatment of hormone depletion, while avoiding the concomitant liability of adverse side effects associated with hormone administration, said medicament comprising 0.01 - 7 mg of hormone.

### DETAILED DESCRIPTION OF THE INVENTION.

Now it has been surprisingly found out that the sex hormones can be administered in very low doses and still be able to achieve significant improvements in subject's well-being. It has been realised that by administering sex hormones in very low levels through oral mucosa it is possible to imitate the natural variation in hormone levels in circulation. The hormone released by the pharmaceutical composition according to the present invention reaches the tissues in a pulse like manner, corresponding to physiological secretion for testis or ovary. Thus the body is exposed for the elevated hormone level only for a short time, which reduces the possibility for negative side effects. The small physiological amount of the hormone that is released also minimise the formation of metabolites, which could cause adverse side effects.

Use of low levels of hormones makes the composition according to the present invention especially suitable for long-term administration, as the risk for negative side effects can be minimised and there is no risk for overdose. The composition is also cheaper to prepare as the amount of active hormone is low in the composition.

In the present context the term sex hormone denotes a natural sex hormone occurring naturally in a healthy human subject. The preferred sex hormones are testosterone and estradiol. These sex hormones can be solubilised by inclusion complexes formed with cyclodextrins and their derivatives. Cyclodextrin derivatives preferably comprise partial methyl, hydroxypropyl or sulfoalkyl ethers, or partial acetyl esters of alpha-, beta- or gamma-cyclodextrins.

According to one embodiment of the invention the sex hormone is testosterone and the amount of the hormone in the pharmaceutical composition is 0.1 - 5 mg, preferably 0.3 - 3 mg, more preferably 0.4 - 2 mg. These low amounts are sufficient to produce a hormone pulse that enhances the overall well-being of the male subjects.

According to another embodiment of the invention the the sex hormone is estradiol and the amount of the hormone is 0.01 - 0.1 mg, preferably 0.015 - 0.05 mg, more preferably 0.02 - 0.04 mg. This embodiment is especially suitable for hormone supplementation to female subjects.

According to one embodiment of the present invention the pharmaceutical composition is in form of a sublingual administration formula. Especially preferred are sublingual administration forms, where the hormone is applied or absorbed on an absorbing matrix, such as absorbing paper. The matrix may or may not disintegrate in water. As the sex hormone is complexed with cyclodextrin derivative, the hormone is highly water soluble and will easily be absorbed through oral mucosa. Naturally other administration forms suitable for oral transmucosal administration can be used.

Preferably the period during which the sex hormone is supplied through oral mucosa into the circulation is as short as possible. By using sublingual administration, a well-defined and controlled entry can be achieved. The area from which absorption of the hormone occurs is highly vascular and does not contain tissues, which would slow down transfer of the hormone into circulation. Any solubilized hormone that may be accidentally swallowed is not transferred into general circulation, but is metabolized by liver. That, in conjunction with the constant formation of saliva, results in ending of the absorption process in a well-defined manner.

According to one embodiment of the invention the pharmaceutical composition is especially suitable for counteracting adverse effects caused by decreasing sex hormone levels at ageing. Low levels of sex hormone, such as testosterone, may result in reduced muscular strength and may cause depression or apathy. Muscular weakness, together with diminished sense of balance may lead to accidental falls, which can thus be prevented by the use of the pharmaceutical composition according to the invention. The present invention is especially suitable for treating signs of ageing in male subjects.

The pharmaceutical composition according to the present invention can also be used to enhance the female sex hormone levels after childbirth. Often a decline in sex hormone level is caused by natural postnatal changes. These low hormone levels may result in postnatal depression or in general indisposition. The present invention can be used to counteract these adverse effects. As the hormone levels employed in the present invention are low, they are safe for the nursing female and the child. The present invention is also suitable for treating the symptoms of female menopause.

According to an embodiment of the present invention the pharmaceutical composition can be used for the normalisation of skin in aging men. Low testosterone levels may cause flabbiness and increased drying of the skin. The composition according to the present invention can be used to prevent or treat this condition.

Sex hormone level in a human is subject to roughly quotidian cycles. Therefore it is natural if supplement comprising the sex hormone in levels according to the invention is administered once or twice a day. Administration can be also three, four or fife times a day, if the single dose is kept is at low level. One of the advantages of the present invention is that the administration can be done in a manner that imitates the body's natural hormone level fluctuation.

The present invention is illustrated by the following examples that should not be considered limiting.

### EXPERIMENTAL PART

### EXAMPLE 1

The purpose of experiment in Example 1 is to ascertain if the above administration of testosterone to man can be performed safely. Adult male produces roughly 25 mg of testosterone during the whole day, and to enter that amount of testosterone into the circulation in a few minutes may have undesired consequences.

For preparation of sublingual pharmaceutical form of testosterone the process delineated in U.S. patent 6,884,790B2 is used. Hydroxypropyl beta-cyclodextrin, pharmaceutical grade, 12 parts, is dissolved in water to form 50% w/w solution, then 1 part of testosterone is added and the suspension stirred for two days. Nearly all testosterone is dissolved at this time and solution is fully clarified by filtration. Testosterone concentration in the solution is determined by spectrophotometry. To prepare the required pharmaceutical form a volume of the solution containing the desired dose of testosterone is deposited on disk of filtration paper about 2 cm in diameter and left to dry fully at room temperature. Small droplet of diluted solution of food dye is spotted on the rim of the disk and left also to dry fully.

Subjects are instructed to insert the disc under tongue and keep it there for three to five minutes, then check whether dye spot disappeared. If that is so, the water-soluble content of the disc is eluted fully, and the disc is discarded.

Few healthy elderly men undergo blood testing. Subjects are administered sublingually 5 mg testosterone using the above specified paper disks. This dose represents more than double of the expected recommended daily dose of testosterone supplementation. The dose is administered in mornings of two subsequent days. Two days after the administration subjects again undergo blood screening testing.

Subjects do not observe any ill effects. Blood tests results do not indicate any untoward effects. Following parameters are evaluated: lipoprotein cholesterol fractions, follicle stimulating hormone (FSH) and luteinizing hormone (LH) concentrations, liver function indicators and sex-hormone binding globulin (SHBG) concentration changes. Conclusion is that to human amount of testosterone envisioned to satisfy the need of one day can be safely administered as a single dose.

### EXAMPLE 2

The purpose of Example 2 is to ascertain bioefficacy of once a day of sublingual dose of testosterone. Of interest are scores of physical stamina, scores of sexual motivation and scores of mood elevation. Since all these parameters are evaluated subjectively, subjects do not know whether disks are placebo or contain hormone. To eliminate individual variation cross over design is used. Each subject self-administers each morning a disc. These discs are of the same kind, i.e. placebo or testosterone containing, for a week. The following week subject receives discs of the other kind.

Subjects score their impressions on scale one to three - one being no change, two weakly felt improvement, and three noticeably felt improvement. Mood elevation score is recorded for period within two hours of the administration, score for physical stamina is recorded after standard exercise performed in afternoon of the administration and scores for sexual motivation apply to 24-hour period after administration.

Scores recorded by subjects are given in the Table below. Subjects, even when given medication in blind manner, can distinguish placebo discs and discs with medication just by their bioeffects. Overall positive feeling of the subject treated with discs containing testosterone tend to increase with the length of the treatment.

It will be appreciated that the present invention can be incorporated in the form of a variety of embodiments, only a few of which are disclosed herein. It will be apparent for the specialist in the field that other embodiments exist and do not depart from the spirit of the invention. Thus, the described embodiments are illustrative and should not be construed as restrictive.

## Claims

1. A pharmaceutical composition adapted for oral transmucosal administration comprising a water-soluble complex of a sex hormone and a cyclodextrin derivative,
**characterised in that**
the amount of sex hormone in the composition is 0.01 - 7 mg.

2. Pharmaceutical composition according to claim 1, **characterised in that** the sex hormone is testosterone and the amount of the hormone is 0.1 - 5 mg, preferably 0.3 - 3 mg.

3. Pharmaceutical composition according to claim 1, **characterised in that** the sex hormone is estradiol and the amount of the hormone is 0.01 - 0.1 mg, preferably 0.015 - 0.05 mg.

4. Pharmaceutical composition according to any of the preceding claims, **characterised in that** the pharmaceutical composition is in form of a sublingual administration formula.

5. Use of a water-soluble complex of a sex hormone and a cyclodextrin derivative for manufacture of an oral transmucosal supplement for treatment of hormone depletion, while avoiding the concomitant liability of adverse side effects associated with conventional hormone administration, said medicament comprising 0.01 - 7 mg of hormone.

6. Use according to claim 5, **characterised in that** the hormone depletion is associated with ageing.

7. Use according to claim 5, **characterised in that** the hormone depletion is caused by natural postnatal changes or menopause.

8. Use according to claim 5 or 6, **characterised in that** the sex hormone is testosterone and the amount of the hormone is 0.1 - 5 mg, preferably 0.3 - 3 mg.

9. Use according to claim 5 or 7, **characterised in that** the sex hormone is estradiol and the amount of the hormone is 0.01 - 0.1 mg, preferably 0.015 - 0.05 mg.

10. Use according to any of the preceding claims 5-9, **characterised in that** the supplement is administered once or twice a day.
